# EUROPÄISCHE PATENTSCHRIFT

(11) **EP 1 786 520 B1**
(45) Veröffentlichungstag und Bekanntmachung des Hinweises auf die Patenterteilung: **09.03.2016**
(21) Anmeldenummer: 05783334.5
(22) Anmeldetag: 04.08.2005
(51) Int. Cl.: A61K 8/81, A61Q 5/04, A61K 8/41

(54) **ZUSAMMENSETZUNG UND VERFAHREN ZUR GLÄTTUNG KERATINHALTIGER FASERN**
COMPOSITION AND METHOD FOR THE SMOOTHING OF FIBRES CONTAINING KERATIN
COMPOSITION ET PROCEDE POUR LISSER DES FIBRES KERATINIQUES

(30) Priorität: 07.09.2004 DE 102004043112
(43) Veröffentlichungstag der Anmeldung: 23.05.2007
(73) Patentinhaber: Henkel AG & Co. KGaA, 40589 Düsseldorf (DE)
(72) Erfinder: MÜLLER, Burkhard, 21075 Hamburg (DE); LUDWIG, Meike, 22085 Hamburg (DE); NEUBUESER, Inge, 22587 Hamburg (DE)
(86) Internationale Anmeldenummer: PCT/EP2005/008438
(87) Internationale Veröffentlichungsnummer: WO 2006/027058

(56) Entgegenhaltungen:
- WO-A-2004/032887
- DE-A1- 10 061 419
- US-A- 4 744 977

## Beschreibung

Die Erfindung betrifft ein auf keratinhaltige Fasern, insbesondere menschliche Haare, oxidativ einwirkendes Mittel, enthaltend neben mindestens einem Oxidationsmittel, eine Wirkstoffkombination aus den Komponenten
(a) einem ausgewählten kationischen Polymer,
(b) mindestens einem Mono- oder Di-(C₈- bis C₃₀)-Alkylammoniumsalz und
(c) mindestens einer quartären Ammoniumverbindung, welche mindestens eine ausgewählte Gruppe am quaternierten Stickstoffatom trägt,
sowie ein Verfahren zur Umformung keratinhaltiger Fasern, in welchem dieses Mittel Anwendung findet, und die Verwendung dieses Mittels zum Fixieren im Rahmen einer Haarumformung.

Als keratinhaltige Fasern können prinzipiell alle tierischen Haare, z.B. Wolle, Roßhaar, Angorahaar, Pelze, Federn und daraus gefertigte Produkte oder Textilien eingesetzt werden. Bevorzugt wird die Erfindung jedoch im Rahmen einer Haarumformung, insbesondere der Glättung krauser menschlicher Haare und daraus gefertigter Perücken, eingesetzt.

Eine dauerhafte Verformung keratinhaltiger Fasern wird üblicherweise derart durchgeführt, dass man die Faser mechanisch verformt und die Verformung durch geeignete Hilfsmittel festlegt. Vor und/oder nach dieser Verformung behandelt man die Faser mit einer keratinreduzierenden Zubereitung. Nach einem Spülvorgang wird die Faser dann in dem sogenannten Fixierschritt mit einer Oxidationsmittelzubereitung behandelt, gespült und nach oder während des Fixierschritts von den Verformungshilfsmitteln (Wicklern, Papilloten) befreit. Wenn als keratinreduzierende Komponente ein Merkaptan, z.B. Ammoniumthioglykolat, verwendet wird, spaltet dieses einen Teil der Disulfid-Brücken des Keratin-Moleküls zu -SH-Gruppen, so dass es zu einer Erweichung der Keratinfaser kommt. Bei der späteren oxidativen Fixierung werden erneut Disulfid-Brücken im Haarkeratin geknüpft, so dass das Keratingefüge in der vorgegebenen Verformung fixiert wird. Alternativ ist es bekannt, zur Haarverformung anstelle der Merkaptane Sulfit zu verwenden. Durch Hydrogensulfit-Lösungen und/oder Sulfit-Lösungen und/oder Disulfit-Lösungen werden Disulfid-Brücken des Keratins in einer Sulfitolyse nach der Gleichung

R-S-S-R + HSO₃⁽⁻⁾ → R-SH +R-S-SO₃⁽⁻⁾

gespalten und auf diese Weise eine Erweichung der Keratinfaser erreicht. Hydrogensulfit-, sulfit- bzw. disulfithaltige Reduktionsmittel weisen nicht den starken Eigengeruch der merkaptanhaltigen Mittel auf. Die Spaltung kann wie zuvor geschildert in einem Fixierschritt mit Hilfe eines Oxidationsmittels unter Bildung von neuen Disulfid-Brücken wieder rückgängig gemacht werden.

Die permanente Glättung keratinhaltiger Fasern wird analog durch den Einsatz von keratinreduzierenden und -oxidierenden Zusammensetzungen erzielt. In einem entsprechenden Verfahren wird das krause Haar entweder auf Wickler mit einem großen Durchmesser von üblicherweise mehr als 15 mm gewickelt oder das Haar unter Einwirkung der keratinreduzierenden Zusammensetzung glattgekämmt. Anstelle des Wicklers ist es auch möglich, die Faser auf ein Glättungsboard glattzulegen. Glättungsboarde sind üblicherweise rechteckige Tafeln z.B. aus Kunststoff. Vorzugsweise ist die Faser dabei mit der keratinreduzierenden Zubereitung benetzt.

Eine weitere Möglichkeit der Haarglättung ist das Glätten mit einem heißen Eisen. Allerdings verändert sich die Struktur der keratinhaltigen Faser bei der Wärmebehandlung des Haars während des Glättens (siehe dazu R. McMullen et al., J. Cosmet. Sci., 1998, 49, 223-244). Dieser Änderung der Faserstruktur sollte durch geeignete Maßnahmen entgegengewirkt werden.

Im allgemeinen haben die bekannten Umformungsverfahren, besonders bei der Glättung, den Nachteil, dass sich die keratinhaltige Faser elektrostatisch auflädt. Darüber hinaus ist das Umformungsergebnis der bekannten Verfahren hinsichtlich des Umformungsgrades und der Gleichmäßigkeit der Umformung verbesserungswürdig. Wenn eine Verbesserung des Umformungsgrades erzielt wird, geht dies meist mit einer verstärkten Schädigung der keratinhaltigen Faser einher.

Aufgabe der Erfindung ist es daher, ein Umformungsverfahren für keratinhaltige Fasern, insbesondere für menschliches Haar, bereitzustellen, das ein verbessertes Umformungsergebnis liefert, die Faser pflegt sowie die Struktur der Faser schont.

In der Druckschrift WO-A1-97/25964 werden leave-on Mittel zur Konditionierung keratinhaltiger Fasern offenbart, die kationische Polymere, insbesondere Polyquaternium-37, enthalten. Die Verwendung dieser Polymere in der erfindunsgemäßen Kombination mit zwei weiteren speziellen kationischen Verbindungen wird nicht erwähnt.

In den Druckschriften EP-A1-1 339 379 und EP-A1-1 280 496 werden wasserstoffperoxidhaltige Mittel mit Polyquaternium-37 offenbart.

In der Druckschrift EP-A1-814 767 wird die Kombination eines quaternären Dialkylammoniumsalzes mit mindestens einer Esterbindung und einem weiteren kationischen Tensid als Konditioniermittel für keratinhaltige Fasern offenbart.

Überraschenderweise wurde gefunden, dass die Aufgabe durch das unten näher erläuterte erfindungsgemäße Mittel gelöst wird. Dieses Mittel dient als Fixiermittel in einem Verfahren zur Umformung keratinhaltiger Fasern, insbesondere menschlichen Haaren.

Ein erster Gegenstand der Erfindung ist ein Mittel, enthaltend neben mindestens einem Oxidationsmittel, eine Wirkstoffkombination aus den Komponenten
(a) einem kationisches Polymer, das mindestens ein Strukturelement der allgemeinen Formel (I) besitzt, 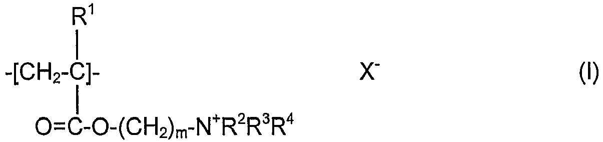 in der
   - R¹ ein Wasserstoffatom oder eine (C₁- bis C₄)-Alkylgruppe ist,
   - R², R³ und R⁴ unabhängig voneinander stehen für (C₁- bis C₄)-Alkylgruppen, (C₂- bis C₄)-Alkenylgruppen oder (C₂- bis C₄)-Hydroxyalkylgruppen,
   - m eine Zahl 1, 2, 3 oder 4 ist und
   - X⁻ ein physiologisch verträgliches organisches oder anorganisches Anion ist,
(b) mindestens einer quartären Ammoniumverbindung der Formel (II)

   (R₅)_{y}(Me)_{(4-y)}N⁺ Y⁻ (II)

   wobei
   R⁵ eine lineare oder verzweigte (C₈- bis C₃₀)-Alkylgruppe,
   y gleich 1 oder 2 ist und
   Y⁻ ein physiologisch verträgliches organisches oder anorganisches Anion ist, und
(c) mindestens einer quartären Ammoniumverbindung, welche mindestens eine Gruppe der Formel (III) enthält,

   R⁶-A¹-A²-A³- (III)

   wobei
   R⁶ für eine gesättigte oder ungesättigte, lineare oder verzweigte, gegebenenfalls substituierte (C₈- bis C₃₀)-Kohlenwasserstoffgruppe steht,
   A¹ für eine Carbonylgruppe oder eine direkte Bindung steht,
   A² für eine direkte Bindung, NH oder ein Sauerstoffatom steht und
   A³ eine (C₂- bis C₄)-Alkylengruppe oder eine (C₂- bis C₄)-Hydroxyalkylengruppe bedeutet, mit den Maßgaben, dass
      A³ an das quartäre Stickstoffatom der quartären Ammoniumverbindung bindet und
      A³ eine (C₂- bis C₄)-Hydroxyalkylengruppe bedeutet, wenn A¹ und A² für eine direkte Bindung stehen.

Die folgenden Beispiele für Substituenten der oben genannten Formeln sind auf die Formeln (I), (II), (III), (c1) und (c2) anzuwenden:
Beispiele für eine (C₁- bis C₄)-Alkylgruppe sind Methyl, Ethyl, Propyl, Isopropyl, n-Butyl, sec-Butyl und tert.-Butyl.
Beispiele für eine (C₈- bis C₃₀)-Alkylgruppe sind n-Octyl, n-Decyl, n-Dodecyl, n-Tetradecyl, n-Hexadecyl, Stearyl, Isostearyl und Eicosyl.
Beispiele für eine (C₁- bis C₃₀)-Alkylgruppe sind die vorgenannten Beispiele für die jeweiligen Alkylgruppen sowie n-Pentyl, eine Isopentyl, n-Hexyl und eine n-Heptylgruppe. Beispiele für eine (C₂- bis C₄)-Alkenylgruppe sind Vinyl, Allyl und Butenyl.
Beispiele für eine (C₂- bis C₄)-Hydroxyalkylgruppe sind eine 2-Hydroxyethyl-, eine 2-Hydroxypropyl, eine 3-Hydroxypropyl- und eine 4-Hydroxybutylgruppe.
Beispiele für eine (C₂- bis C₄)-Alkylengruppe ist Ethylen, Propan-1,2-diyl, Propan-1,3-diyl, und Butan-1,4-diyl.
Beispiele für eine (C₂- bis C₄)-Hydroxyalkylengruppe ist 2-Hydroxyethylen, 1-Hydroxy-propan-1,2-diyl, 1-Hydroxy-propan-1,3-diyl und 2-Hydroxy-propan-1,3-diyl.

Das in dem erfindungsgemäßen Mittel enthaltene Oxidationsmittel besitzt ein solches Redox-Potential, dass zwei Mercaptogruppen unter Bildung einer Disulfidbrücke oxidiert werden können. Ein bevorzugtes Oxidationsmittel wird ausgewählt aus z.B. Natriumbromat, Kaliumbromat oder Wasserstoffperoxid. Es ist besonders bevorzugt, Wasserstoffperoxid als Oxidationsmittel zu verwenden. Zur Stabilisierung wäßriger Wasserstoffperoxidzubereitungen können zusätzlich übliche Stabilisatoren zugesetzt werden. Der pH-Wert der wäßrigen H₂O₂-Zubereitungen, die üblicherweise etwa 0,5 bis 3,0 Gew.-% H₂O₂ enthalten, liegt bevorzugt bei 2 bis 6. Enthält das erfindungsgemäße Mittel Bromat als Oxidationsmittel, so ist dies üblicherweise in Konzentrationen von 1 bis 10 Gew.-% enthalten und der pH-Wert der Lösungen wird auf 4 bis 7 eingestellt.

Es sind solche kationischen Polymere mit mindestens einer Struktureinheit gemäß Formel (I) erfindungsgemäß bevorzugt, für die mindestens eine der folgenden Bedingungen gilt:
R¹ steht für eine Methylgruppe
R², R³ und R⁴ stehen für Methylgruppen
m hat den Wert 2.

Als physiologisch verträgliches organisches oder anorganisches Gegenionen X⁻ gemäß Formel (I) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Liegt das in den erfindungsgemäßen Mitteln enthaltene kationische Polymer als Copolymer vor, so bestehen diese im wesentlichen aus den in Formel (I) aufgeführten Strukturelementen sowie aus nichtionogenen Co-Monomereinheiten. Copolymere mit Strukturelementen gemäß Formel (I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere weiter unten beschrieben, vernetzt sein. Ein erfindungsgemäß bevorzugtes Copolymer ist das vernetzte Acrylamid/Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Homopolymere aus einem Strukturelement gemäß Formel (I) sind besonders bevorzugte kationische Polymere.

Ein besonders bevorzugtes kationisches Polymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.
Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Rheocare^{®} CTH (E) bzw. Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecylpolyoxypropylen-polyoxyethylen-ether (INCI-Bezeichnung: PPG-1-Trideceth-6)) im Handel erhältlich.

Die erfindungsgemäßen Mittel enthalten das kationische Polymer mit mindestens einem Strukturelement der Formel (I) bevorzugt in einer Menge von 0,01 bis 10 Gew.-%, besonders bevorzugt von 0,1 bis 5 Gew.-%, jeweils bezogen auf das Gewicht des Mittels.

Für die Komponente (b) des erfindungsgemäßen Mittels steht der Rest R⁵ der Formel (II) bevorzugt für eine Alkylgruppe mit 10 bis 22 Kohlenstoffatomen, besonders bevorzugt mit 10 bis 18 Kohlenstoffatomen.

Die in den erfindungsgemäßen Mitteln als Komponente (b) enthaltenen quartären Ammoniumverbindungen der Formel (II) sind bevorzugt Halogenide, insbesondere Chloride und Bromide.

Bevorzugt sind als Verbindung der Formel (II) Salze, insbesondere Halogenide, des Dicetyldimethylammoniums, Distearyldimethylammoniums (z.B. Dioctadecyldimethylammonium Chlorid als Genamin^{®} DSAc der Firma Clariant), Cetyltrimethylammoniums (z.B. Hexadecyltrimethylammonium Chlorid als Dehyquart^{®} A der Firma Cognis), oder des Stearyltrimethylammoniums.

Die quartären Ammoniumverbindungen (c) enthalten gesättigte oder ungesättigte, lineare oder verzweigte, gegebenenfalls substituierte (C₈- bis C₃₀)-Kohlenwasserstoffgruppen. Darunter sind zunächst Kohlenwasserstoffgruppen zu verstehen, welche 8 bis 30 Kohlenstoffatome enthalten, die ein lineares oder verzweigtes, gesättigtes oder ungesättigtes Kohlenstoffrückgrat bilden. Dieses Kohlenstoffrückgrat wird nicht von Heteroatomen unterbrochen. Heteroatome sind Atome, welche von Kohlenstoff und Wasserstoff verschieden sind, insbesondere Stickstoff, Sauerstoff und Schwefel. Es sind erfindungsgemäß solche Kohlenwasserstoffgruppen mitumfaßt, welche Heteroatom-haltige Gruppen als Substituenten für Wasserstoffatome tragen. Es ist erfindungsgemäß bevorzugt, dass die gesättigten oder ungesättigten, linearen oder verzweigten (C₈ bis C₃₀)-Kohlenwasserstoffgruppen 0 bis 4 solcher Heteroatom-haltigen Gruppen tragen, insbesondere 0 bis 2, besonders bevorzugt 1 oder 0. Bevorzugte erfindungsgemäße gesättigte oder ungesättigte, lineare oder verzweigte (C₈ bis C₃₀)-Kohlenwasserstoffgruppen sind lineare oder verzweigte (C₈-C₃₀)-Alkylgruppen (Beispiele dafür wurden zuvor definiert), lineare oder verzweigte (C₈-C₃₀)-Alkenylgruppen (beispielsweise 9-Octadecen-1-yl, 9,12-Octadecadien-1-yl, 9,12,15-Octadecatrien-1-yl, 13-Docosen-1-yl), Hydroxy-(C₈-C₃₀)-alkylgruppen (beispielsweise 2-Hydroxydecyl, 2-Hydroxydodecyl, 2-Hydroxytetradecyl, 2-Hydroxyhexadecyl und 2-Hydroxyoctadecyl) und Oligohydroxy-(C₈-C₃₀)-alkylgruppen mit bevorzugt 2 bis 4 Hydroxylgruppen.

Bei der Auswahl der Verbindungen (c) ist es bevorzugt, wenn die Gruppe gemäß Formel (III) ausgewählt wird, aus
- einer ω-[(C₈- bis C₃₀)-Alkanoyloxy]-(C₂- bis C₄)-alkylgruppe (R⁶ = (C₈- bis C₃₀)-Alkyl, A¹ = Carbonyl, A² = Sauerstoffatom, A³ = (C₂- bis C₄)-Alkylengruppe),
- einer ω-[(C₈- bis C₃₀)-Alkylamido]-(C₂- bis C₄)-alkylgruppe (R⁶ = (C₈- bis C₃₀)-Alkyl, A¹ = Carbonyl, A² = Gruppe NH, A³ = (C₂- bis C₄)-Alkylengruppe),
- einer (C₁₀- bis C₃₄)-Hydroxyalkylgruppe (entweder: R⁶ = (C₈- bis C₃₀)-Alkyl, A¹ = direkte Bindung, A² = direkte Bindung, A³ = (C₂- bis C₄)-Hydroxyalkylengruppe oder: R⁶ = (C₈- bis C₃₀)-Hydroxyalkyl oder Oligohydroxy-(C₈- bis C₃₀)-alkylgruppe, A¹ = direkte Bindung, A² = direkte Bindung, A³ = (C₂- bis C₄)-Alkylengruppe.

Beispiele für ω-[(C₈- bis C₃₀)-Alkanoyloxy]-(C₂- bis C₄)-alkylgruppen sind die bevorzugten 2-[(C₈- bis C₃₀)-Acyloxy]ethylgruppen, insbesondere 2-(Dodecanoyloxy)ethyl, 2-(Tetradecanoyloxy)ethyl, 2-(Hexadecanoyloxy)ethyl und 2-(Octadecanoyloxy)ethyl.

Es sind bevorzugt solche quartären Ammoniumverbindungen (c) in dem erfindungsgemäßen Mittel enthalten, ausgewählt aus der Gruppe, bestehend aus Komponenten (c) mit einer und zwei Gruppen der Formel (III).

Es ist erfindungsgemäß bevorzugt, dass die quartären Ammoniumverbindungen (c) in einer Menge von 0,05 bis 5 Gew.-%, bezogen auf das gesamte Mittel, in dem erfindungsgemäßen Mittel enthalten sind.

Die quartären Ammoniumverbindungen (c) enthaltend genau eine Gruppe der vorgenannten Formel (III) werden bevorzugt ausgewählt aus Verbindungen der Formel (c1) 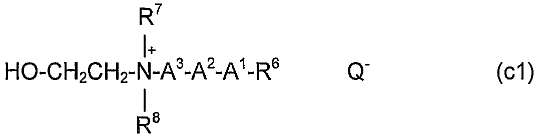 worin
R⁷ und R⁸ unabhängig voneinander ein Wasserstoffatom, eine (C₂- bis C₃₀)-Acylgruppe oder eine lineare oder verzweigte (C₁- bis C₃₀)-Alkylgruppe bedeutet,
R⁶ eine lineare oder verzweigte (C₈- bis C₃₀)-Alkylgruppe oder eine lineare oder verzweigte (C₈- bis C₃₀)-Alkenylgruppe bedeutet,
A¹ eine direkte Bindung oder eine Carbonylgruppe bedeutet,
A² eine direkte Bindung, eine Gruppe NH oder ein Sauerstoffatom bedeutet,
A³ eine (C₂- bis C₄)-Alkylengruppe oder eine (C₂- bis C₄)-Hydroxyalkylengruppe bedeutet und
Q⁻ ein physiologisch verträgliches organisches oder anorganisches Anion ist,
mit der Maßgabe, dass A³ eine (C₂- bis C₄)-Hydroxyalkylengruppe bedeutet, wenn A¹ und A² für eine direkte Bindung stehen.

Als physiologisch verträgliches organisches oder anorganisches Gegenionen Q⁻ gemäß Formel (c1) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfat und Halogenidionen, insbesondere Chlorid.

Es sind solche Verbindungen gemäß Formel (c1) erfindungsgemäß bevorzugt, für die mindestens eine der folgenden Bedingungen gilt:
- A¹ steht für eine Carbonylgruppe, wenn A² für ein Sauerstoffatom oder eine Gruppe NH steht.
- R⁷ und R⁸ stehen für eine (C₁- bis C₄)-Alkylgruppe, insbesondere eine Methylgruppe.

Es gelten die Beispiele für die Reste gemäß Formel (c1), wie sie für die jeweiligen Reste bzw. Gruppen der Formeln (I), (II) und (III) genannt wurden.
Beispiele für eine (C₁-bis C₄)-Alkylengruppe sind Methylen, Ethylen, Propan-1,2-diyl, Propan-1,3-diyl, und Butan-1,4-diyl.
Beispiele für (C₈- bis C₃₀)-Acylgruppen sind Octanoyl, Decanoyl, Dodecanoyl, Tetradecanoyl, Hexadecanoyl, Octadecanoyl und Eicosanoyl.

Die quartären Ammoniumverbindungen (c) enthaltend zwei Gruppen der vorgenannten Formel (III) werden generell bevorzugt ausgewählt aus Verbindungen mit der Formel (c2) 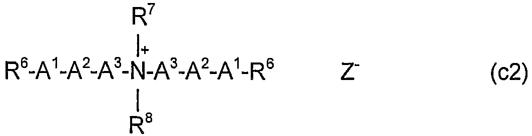 worin
- R⁷ und R⁸ unabhängig voneinander für ein Wasserstoffatom, eine (C₁- bis C₄)-Alkylgruppe oder (C₂- bis C₄)-Hydroxyalkylgruppe stehen,
- R⁶ eine lineare oder verzweigte (C₈- bis C₃₀)-Alkylgruppe, lineare oder verzweigte (C₈- bis C₃₀)-Alkenylgruppe, eine Hydroxy-(C₈- bis C₃₀)-alkylgruppe oder eine Oligohydroxy-(C₈- bis C₃₀)-alkylgruppe bedeutet,
- A¹ eine Carbonylgruppe bedeutet,
- A² eine Gruppe NH oder ein Sauerstoffatom bedeutet,
- A³ eine (C₂- bis C₄)-Alkylengruppe, eine (C₂- bis C₄)-Hydroxyalkylengruppe bedeutet und
- Z⁻ ein physiologisch verträgliches organisches oder anorganisches Anion ist.

Es gelten die Beispiele für die Reste gemäß Formel (c2), wie sie für die jeweiligen Reste bzw. Gruppen der Formeln (I), (II), (III) und (c1) genannt wurden.

Es sind solche Verbindungen gemäß Formel (c2) erfindungsgemäß bevorzugt, für die mindestens eine der folgenden Bedingungen gilt:
- R⁷ und R⁸ stehen bevorzugt unabhängig voneinander für ein Wasserstoffatom, eine Methylgruppe oder eine 2-Hydroxyethylgruppe.
- Wenn R⁷ und R⁸ gleich sind, stehen sie bevorzugt für eine (C₁- bis C₄)-Alkylgruppe, besonders bevorzugt für eine Methylgruppe.
- Es ist bevorzugt, dass A³ ausgewählt wird aus Ethylen, Propan-1,3-diyl, Propan-1,2-diyl, 2-Hydroxypropan-1,3-diyl.

Als physiologisch verträgliches organisches oder anorganisches Gegenionen Z gemäß Formel (c2) kommen beispielsweise Halogenidionen, Sulfationen, Phosphationen, Methosulfationen sowie organische Ionen wie Lactat-, Citrat-, Tartrat- und Acetationen in Betracht. Bevorzugt sind Methosulfat und Halogenidionen, insbesondere Chlorid.

Die Verbindungen der erfindungsgemäßen Komponente (c) werden bevorzugt ausgewählt aus der Gruppe, die gebildet wird aus
- quaternierten Estersalzen von Fettsäuren mit Triethanolamin, sogenannte Esterquats, insbesondere den Salzen des N,N-Di(2-(dodecanoyloxy)-ethyl)dimethylammoniums, N,N-Di(2-(tetradecanoyloxy)ethyl)dimethyl-ammoniums, N,N-Di(2-(hexadecanoyloxy)ethyl)dimethylammoniums, N,N-Di(2-(hexadecanoyloxy)propyl)dimethylammoniums, N,N-Di(2-(octadecanoyloxy)-ethyl)dimethylammoniums mit einem physiologisch verträglichen, organisch oder anorganischen Anion. Bevorzugte Anionen sind Alkylsulfate, wie z.B. Methylsulfat, und Halogenide, wie Chlorid und Bromid. Solche Produkte werden beispielsweise unter den Warenzeichen Stepantex^{®}, Dehyquart^{®} und Armocare^{®} vertrieben. Die Produkte Armocare^{®} VGH-70, ein N,N-Di(2-Hexadecanoyloxyethyl)dimethylammoniumchlorid, sowie Dehyquart^{®} L80 (INCI-Bezeichnung: Dicocoylethyl Hydroxyethylmonium Methosulfate), Dehyquart^{®} F-75, Dehyquart^{®} C-4046, und Dehyquart^{®} AU-35 sind Beispiele für solche Esterquats. Zur Herstellung können auch Fettsäureschnitte, z.B. aus Talg wie beispielsweise Rindertalg gewonnene Talgfettsäuren, verwendet werden (INCI-Bezeichnungen: Ditallowoylethyl Dimonium Methosulfate, Ditallowoyl PG-dimonium Chloride),
- Salzen des N,N-Dimethyl-N-(2-hydroxyethyl)-N-(2-hydroxyhexadecyl)-ammoniums. Bevorzugte Halogenide sind Chlorid und Bromid. Diese werden beispielsweise unter der Handelsmarke Dehyquart^{®} E (INCI-Bezeichnung: Aqua (Water), Hydroxycetyl Hydroxyethyl Dimonium Chloride) von der Firma Cognis vertrieben,
- quaternierte Amidsalze von Fettsäuren mit Diaminen, wie z.B. Salzen des N-(13-Docosen)amidopropyl-N-2-hydroxyethyl-N,N-dimethylammoniums (INCI-Bezeichnung: Hydroxyethyl Erucamidopropyl Dimonium Chloride) oder des N-Docosylamidopropyl-N-2-hydroxyethyl-N,N-dimethylammoniums, welches unter dem Handelsnamen Incroquat^{®} Behenyl HE (INCI-Bezeichnung: Hydroxyethyl Behenamidopropyl Dimonium Chloride) von der Firma Croda Inc. vertrieben wird.

Die kationischen Polymere (a) und die quaternierten Ammoniumverbindungen (b) werden bevorzugt in einem Bereich des Stoffmengenverhältnisses Stoffmenge (a) zu Stoffmenge (b) von 0,2 zu 1 bis 5 zu 1, besonders bevorzugt im Bereich von 1 zu 1 bis 3 zu 1 in den erfindungsgemäßen Mitteln verwendet.
Die kationischen Polymere (a) und die quaternierten Ammoniumverbindungen (c) werden bevorzugt in einem Bereich des Stoffmengenverhältnisses Stoffmenge (a) zu Stoffmenge (c) von 0,2 zu 1 bis 5 zu 1, besonders bevorzugt im Bereich von 1 zu 1 bis 3 zu 1 in den erfindungsgemäßen Mitteln verwendet.

Die erfindungsgemäßen Mittel können zusätzlich Silikone enthalten. Erfindungsgemäß verwendbare Silikone sind bevorzugt lineare, cyclische oder verzweigte Silikone, ausgewählt aus den Typen der Cyclomethicone, Dimethiconole, Dimethiconcopolyole, Amodimethicone, Trimethylsilylamodimethicone und Phenyltrimethicone. Diese Silikontypen sind dem Fachmann unter der Nomenklatur der Cosmetic, Toiletry and Fragrance Association (CTFA) bekannt und in: M.D. Berthiaume, Society of the Cosmetic Chemists Monograph Series, "Silicones in Hair Care", Ed.: L. D. Rhein, Hrsg.: Society of the Cosmetic Chemists, 1997, Kapitel 2 offenbart, worauf an dieser Stelle explizit verwiesen wird. Polysiloxane, wie Dialkyl- und Alkylarylsiloxane, beispielsweise Dimethylpolysiloxan und Methylphenylpolysiloxan, sowie deren alkoxylierte Analoga, mit Hydroxylgruppen terminierte Analoga und quaternierte Analoga, sowie cyclische Siloxane. Dabei sind die Silikone mit den INCI-Bezeichnungen Dimethicone, PEG-12 Dimethicone, PEG/PPG-18/18 Dimethicone, Cyclomethicone, Dimethiconol, Quaternium-80 und Amodimethicone sowie deren Gemische besonders bevorzugt.

Beispiele für solche Silikone sind die von Dow Corning unter den Bezeichnungen DC 190 (INCI-Bezeichnung: PEG/PPG-18/18 Dimethicone), DC 193 (INCI-Bezeichnung: PEG-12 Dimethicone), DC 200, DC1401 (INCI-Bezeichnung: Cyclomethicone, Dimethiconol) und DC 1403 (INCI-Bezeichnung: Dimethicone, Dimethiconol) vertriebenen Produkte sowie die Handelsprodukte DC 244 (INCI-Bezeichnung: Cyclomethicone), DC 344 (INCI-Bezeichnung: Cyclomethicone) und DC 345 (INCI-Bezeichnung: Cyclomethicone) von Dow Corning, Q2-7224 (Hersteller: Dow Corning; ein stabilisiertes Trimethylsilylamodimethicon, Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil Quat 3270 und 3272 (Hersteller: Th. Goldschmidt; diquaternäre Polydimethylsiloxane, INCI-Bezeichnung: Quaternium-80).

Die Silikone sind bevorzugt in den Mengen von 0,1 bis 10 Gew.-%, besonders bevorzugt von 0,3 bis 5 Gew.-%, jeweils bezogen auf das Gewicht des Mittels enthalten.

Die erfindungsgemäßen Mittel können zusätzlich Proteinhydrolysate enthalten. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (RITA Corp.), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Ein bevorzugtes Proteinhydrolysat ist das Seidenproteinhydrolysat (Promois^{®} Silk 720, Promois^{®} Silk 1000).

Erfindungsgemäß ist ebenso die Verwendung von Proteinhydrolysaten pflanzlichen Ursprungs möglich, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.

Wenngleich der Einsatz der Proteinhydrolysate als solche bevorzugt ist, können an deren Stelle gegebenenfalls auch anderweitig erhaltene Aminosäuregemische oder einzelne Aminosäuren und Aminosäurederivate wie beispielsweise Arginin, Asparagin, Asparaginsäure, Citrullin, Histidin, Ornithin, Lysin und Pyrroglutaminsäure eingesetzt werden. Die Aminosäuren können sowohl als freie Aminosäure, als auch als Salze, z. B. als Hydrochloride oder der Alkali, Erdalkali oder Ammoniumsalze eingesetzt werden. Weiterhin haben sich auch Oligopeptide aus durchschnittlich 2-3 Aminosäuren, die einen hohen Anteil (> 50 %, insbesondere > 70 %) an den genannten Aminosäuren haben, als erfindungsgemäß einsetzbar erwiesen.

Erfindungsgemäß besonders bevorzugt sind Arginin, Asparagin, Asparaginsäure sowie deren Salze und Oligopeptide bzw. Hydrolysate, welche reich an den genannten bevorzugten Aminosäuren sind. Ganz besonders bevorzugt sind Asparagin und Asparaginsäure sowie deren Salze bzw. Hydrolysate.

Weiterhin können die erfindungsgemäßen Mittel mindestens einen oberflächenaktiven Stoff aus der Gruppe der anionischen, amphoteren, zwitterionischen und nichtionischen Tenside enthalten. Die Tenside haben unter anderem die Aufgabe, die Benetzung der Keratinoberfläche durch die Behandlungslösung zu fördern.

Als anionische Tenside eignen sich in erfindungsgemäßen Zubereitungen prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 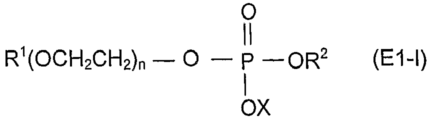 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C1 bis C4 - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 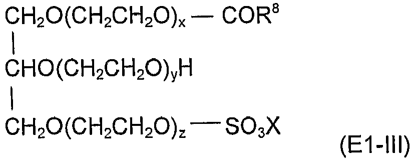 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus C₈ - C₃₀ - Fettalkoholen mit Proteinhydrolysaten und/oder Aminosäuren und deren Derivaten, welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremonoalkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine - COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.
   Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoaikohoie (DP = 1 bis 3). Der Alkyl-bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

- Zuckertenside vom Typ der Fettsäure-N-alkylpolyhydroxyalkylamide, ein nichtionisches Tensid der Formel (E4-III), 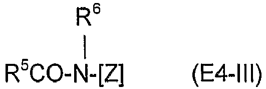 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Hinsichtlich der Verfahren zu ihrer Herstellung sei auf die US-Patentschriften US 1,985,424, US 2,016,962 und US 2,703,798 sowie die Internationale Patentanmeldung WO 92/06984 verwiesen. Eine Übersicht zu diesem Thema von H.Kelken-berg findet sich in Tens. Surf. Det. 25, 8 (1988). Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

   R⁷CO-NR⁸-CH₂-(CHOH)₄CH₂OH (E4-IV)

   Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside in den erfindungsgemäßen Mitteln enthalten sein. Diese können in den erfindungsgemäßen Zusammensetzungen bevorzugt in Mengen von 0,1 bis 20 Gew.-%, bezogen auf die jeweilige gesamte Zusammensetzung, enthalten sein. Mengen von 0,5 bis 15 Gew.-% sind besonders bevorzugt, und ganz besonders bevorzugt sind Mengen von 0,5 bis 7,5 Gew.%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden in Mengen von 0,1 bis 45 Gew.%, bevorzugt 0,5 bis 30 Gew.% und ganz besonders bevorzugt von 0,5 bis 25 Gew.%, bezogen auf die jeweilige gesamte erfindungsgemäß verwendete Zusammensetzung, eingesetzt.

In einer weiteren Ausführungsform können in den erfindungsgemäßen Mitteln Emulgatoren verwendet werden. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Weiterführende Definitionen und Eigenschaften von Emulgatoren finden sich in "H.-D.Dörfler, Grenzflächen- und Kolloidchemie, VCH Verlagsgesellschaft mbH. Weinheim, 1994". Erfindungsgemäß verwendbare Emulgatoren sind beispielsweise
- Anlagerungsprodukte von 4 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 bis 25 Gew.-%, insbesondere 0,1 bis 3 Gew.-%, bezogen auf die jeweilige gesamte Zusammensetzung.

Bevorzugt können die erfindungsgemäßen Mittel mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Die erfindungsgemäßen Mittel enthalten bevorzugt mindestens einen linearen oder verzweigten, gesättigten oder ungesättigten Fettalkohol. Als Fettalkohole können eingesetzt werden Fettalkohole mit C₆-C₃₀-, bevorzugt C₁₀-C₂₂- und ganz besonders bevorzugt C₁₂-C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole werden in Mengen von 0,1 bis 20 Gew.-%, bezogen auf die gesamte Zubereitung, bevorzugt in Mengen von 0,1 bis 10 Gew.-% eingesetzt.

Die erfindungsgemäßen Mittel können zusätzlich eine viskositätserhöhende Verbindung, im weiteren als Verdickungsmittel bezeichnet, enthalten.

Erfindungsgemäß einsetzbare Verdickungsmittel sind beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol, sowie viskositätserhöhende Polymere auf Polyacrylat-Basis wie sie beispielsweise unter den Handelsnamen Pemulen^{®}, Aculyn^{®} und Carbopol^{®} vertrieben werden. Desweiteren wird bevorzugt eine Mischung aus Diestern von 1,2-Propylenglykol mit Fettsäuren, beispielsweise der Verdicker mit der INCI-Bezeichnung Propylene Glycol Dicaprylate / Dicaprate, in den erfindungsgemäßen Mitteln eingesetzt.

Weiterhin können folgende Verbindungen in den erfindungsgemäßen Mitteln enthalten sein:
- lineare und/oder verzweigte Fettsäuren, bevorzugt C₂-C₃₀-Fettsäuren, besonders bevorzugt C₄-C₁₈ Fettsäuren, am meisten bevorzugt C₆-C₁₀-Fettsäuren und/oder deren physiologisch verträglichen Salzen; Weitere Beispiele sind Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Milchsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure,
- Polyhydroxyverbindungen; hierbei sind insbesondere zu nennen
   - Zucker mit 5 und/oder 6 Kohlenstoffatomen, insbesondere als Mono- und/oder Oligosaccharide, beispielsweise Glucose, Fructose, Galactose, Lactose, Arabinose, Ribose, Xylose, Lyxose, Allose, Altrose, Mannose, Gulose, Idose, Talose und Sucrose und/oder deren Derivate, z.B. Etherderivate, Aminoderivate und/oder Acetylderivate wie acetylierte Glucose, z.B. Tetraacetylglucose, Pentaacetylglucose und/oder 2-Acetamido-2-desoxyglucose. Bevorzugte Zuckerbausteine sind Glucose, Fructose, Galactose, Allose, Lactose, Arabinose und Sucrose; Glucose, Galactose und Lactose sind besonders bevorzugt;
   - Onsäuren, insbesondere Gluconsäure, Glucuronsäure;
   - Polyole, wie z.B. Glucamine, Glycerin, Mono- oder Diglyceride, 2-Ethyl-1,3-hexandiol, 2-Hydroxymethylpropantriol, Glycole wie Ethylenglykol, Diethylenglykol, Triethylenglykol, Propylenglykol, Dipropylenglykol, 1,3-Butandiol;
   - Polyhydroxysäuren, wie z.B. Pentahydroxyhexansäure, Tetrahydroxypentansäure und/oder deren Derivate, wie z.B. Ether, Ester und/oder Amide, z.B. Pentahydroxyhexansäureamid und/oder deren physiologisch verträglichen Salzen; weitere Beispiele: Zitronensäure, Äpfelsäure oder Weinsäure.
- Pantolacton
- Panthenol und/oder dessen Derivate;
- weitere Vitamine, wie z.B. Vitamin B6, C und/oder E und/oder deren Derivate;
- Hydroxysäuren, wie z.B. α-, β-Hydroxyfettsäuren bzw. Ketofettsäuren und/oder deren physiologisch verträglichen Salzen; wie beispielsweise Salicylsäure oder Milchsäure, Glyoxylsäure, Glycolsäure.
- wasserlösliche Polymere festigender Wirkung, z. B. Polyvinylpyrrolidon, Vinylacetat/Crotonsäure-Copolymere,
- Antischuppenwirkstoffe wie z.B. Picrotone Olamine, Zink Omadine,
- Wirkstoffe wie Allantoin, Pyrrolidoncarbonsäuren, Pflanzenextrakte ,
- pH-Einstell- und Puffermittel wie z.B. Citronensäure/Natriumcitrat, Ammoniumcarbonat, Ammoniumhydrogencarbonat, Guanidincarbonat, Ammoniak, Natriumhydroxyd,
- Komplexbildner wie EDTA, NTA, Organophosphonsäuren, Dipicolinsäure
- Lichtschutzmittel (UV-Absorber)
- Öl-, Fett- und Wachskomponenten, bevorzugt in emulgierter Form,
- Farbstoffe, Trübungs- und Perlglanzmittel sowie
- gegebenenfalls Aerosol-Treibgase

Ein zweiter Gegenstand der Erfindung ist ein Verfahren zur Umformung, insbesondere zur Glättung, keratinhaltiger Fasern, insbesondere menschlicher Haare, in welchem
(i) die Fasern unter Zuhilfenahme von Verformungshilfsmitteln nach, vor oder während Schritt (ii) verformt werden,
(ii) eine wäßrige Zusammensetzung, enthaltend mindestens eine keratinreduzierende Verbindung, auf die Fasern aufgetragen wird,
(iii) die Fasern nach einer Einwirkzeit Z1, gespült und gegebenenfalls getrocknet werden,
(iv) anschließend ein Mittel des ersten Erfindungsgegenstandes auf die Fasern aufgetragen und nach einer Einwirkzeit Z2 wieder abgespült wird.

Verformungshilfsmittel im Sinne des erfindungsgemäßen Verfahrens können
- z.B. Lockenwickler oder Papilloten im Falle einer Dauerwelle,
- oder Hilfsmittel für eine mechanische Glättung, wie ein Kamm oder eine Bürste, ein Glättungsboard oder ein beheizbares Glättungseisen im Falle einer Haarglättung sein.

Wenn die Verformungshilfsmittel, beispielsweise Wickler, im Rahmen eines Dauerwellverfahrens für einen längeren Zeitraum an der Faser befestigt werden, so ist es zweckmäßig, diese Verformungshilfsmittel vor Schritt (iii) oder nach Schritt (iv) zu entfernen. Es kann in diesem Zusammenhang vorteilhaft sein, die Verformungshilfsmittel während des Schritts (iv) im Haar zu belassen, sie danach zu entfernen und danach Schritt (iv) als sogenannten Nachfixierschritt (v) zu wiederholen.

In einer bevorzugten Ausführungsform der Erfindung werden die keratinhaltigen Fasern vor dem Schritt (i) angefeuchtet. Dies kann durch Besprühen der Fasern mit einer Flüssigkeit, bevorzugt mit Wasser, geschehen. Bevorzugterweise werden die Fasern vor Schritt (i) mit einem herkömmlichen Shampoo shampooniert, gespült und dann mit einem Handtuch frottiert. Nach Abschluß des Frottierschritts bleibt eine fühlbare Restfeuchtigkeit im Haar zurück.

Es ist bevorzugt, im Rahmen einer Haarglättung die Fasern während der Einwirkzeit Z1 aus Schritt (ii) mechanisch, insbesondere durch Kämmen oder mit Hilfe eines Glättungsboardes, zu glätten.

Unter einer mechanischen Glättung wird erfindungsgemäß eine Streckung der krausen Faser entlang ihrer längsten räumlichen Ausdehnung verstanden.

Die Einwirkzeit Z1 beträgt bevorzugt 5-60 Minuten, besonders bevorzugt 10-30 Minuten. Die Einwirkzeit Z2 beträgt bevorzugt 1-30 Minuten, besonders bevorzugt 5-20 Minuten.

Eine wäßrige Zusammensetzung im Sinne der Erfindung enthält mindestens 50 Gew.% Wasser bezogen auf das Gewicht der gesamten Zusammensetzung. Diese wäßrige Zusammensetzung kann in verschiedenen Formen, beispielsweise als Lotion, Öl-in-Wasser-Emulsion oder Wasser-in-ÖI-Emulsion, vorliegen.

Die in der wäßrigen Zusammensetzung des Schritts (ii) enthaltenen keratinreduzierenden Verbindungen werden bevorzugt ausgewählt aus Verbindungen mit mindestens einer Thiolgruppe sowie deren Derivate, aus Sulfiten, Hydrogensulfiten und Disulfiten.

Verbindungen mit mindestens einer Thiolgruppe sowie deren Derivate sind beispielsweise Thioglykolsäure, Thiomilchsäure, Thioäpfelsäure, Phenylthioglykolsäure, Mercaptoethansulfonsäure sowie deren Salze und Ester (wie z.B. Isooctylthioglycolat und Isopropylthioglycolat), Cysteamin, Cystein, Bunte Salze und Salze der schwefligen Säure. Bevorzugt geeignet sind die Monoethanolammoniumsalze oder Ammoniumsalze der Thioglykolsäure und/oder der Thiomilchsäure sowie die freien Säuren. Diese werden in der wässrigen Zusammensetzung bevorzugt in Konzentrationen von 0,5 bis 2,0 mol/kg bei einem pH-Wert von 5 bis 12, insbesondere von 7 bis 9,5, eingesetzt. Zur Einstellung dieses pH-Wertes enthalten die wässrigen Zusammensetzungen üblicherweise Alkalisierungsmittel wie Ammoniak, Alkali- und Ammonium-carbonate und - hydrogencarbonate oder organische Amine wie Monoethanolamin.

Beispiele für keratinreduzierende Verbindungen der Disulfite, die in der wässrigen Zusammensetzung enthaltenen sein können, sind Alkalidisulfite, wie z.B. Natriumdisulfit (Na₂S₂O₅) und Kaliumdisulfit (K₂S₂O₅), sowie Magnesiumdisulfit und Ammoniumdisulfit ((NH₄)₂S₂O₅). Ammoniumdisulfit kann dabei erfindungsgemäß bevorzugt sein. Beispiele für keratinreduzierende Verbindungen der Hydrogensulfite, die in der wässrigen Zusammensetzung enthaltenen sein können, sind Hydrogensulfite als Alkali-, Magnesium-, Ammonium- oder Alkanolammonium-Salz auf Basis eines C₂-C₄-Mono-, Di- oder Trialkanolamins. Ammoniumhydrogensulfit kann dabei ein besonders bevorzugtes Hydrogensulfit sein. Beispiele für keratinreduzierende Verbindungen der Sulfite, die in der wässrigen Zusammensetzung enthalten sein können, sind Sulfite als Alkali-, Ammonium- oder Alkanolammonium-Salz auf Basis eines C₂-C₄-Mono-, Di- oder Trialkanolamins. Ammoniumsulfit ist dabei bevorzugt. Der pH-Wert der wässrigen Zusammensetzung wird bei Verwendung von Sulfit und/oder Disulfit und/oder Hydrogensulft bevorzugt auf einen Wert im Neutralbereich von pH 5 bis 8, bevorzugt von pH 6 bis 7,5 eingestellt.

Bevorzugte C₂-C₄-Alkanolamine sind erfindungsgemäß 2-Aminoethanol (Monoethanolamin) und N,N,N-Tris(2-hydroxyethyl)amin (Triethanolamin). Monoethanolamin ist ein besonders bevorzugtes C₂-C₄-Alkanolamin, das insbesondere in einer Menge von 0,2 bis 6 Gew.-% bezogen auf die gesamte wässrige Zusammensetzung, eingesetzt wird.

Die keratinreduzierende Verbindung wird bevorzugt in einer Menge von 5 bis 20 Gew.-%, bezogen auf die gesamte wässrige Zusammensetzung, eingesetzt.

Darüberhinaus kann die wässrige Zusammensetzung weitere Komponenten enthalten, die die Wirkung der keratinreduzierenden Verbindung auf das Keratin fördern. Solche Komponenten sind z.B. Quellmittel für keratinhaltige Fasern wie z.B. C₁-C₆-Alkohole und wasserlösliche Glykole oder Polyole wie z.B. Glycerin, 1,2-Propylenglykol oder Sorbit und Harnstoff oder Harnstoffderivate wie z.B. Allantoin und Guanidin sowie Imidazol und dessen Derivate. Eine bevorzugte weitere Komponente ist 1,2-Propylenglykol, insbesondere in einer Menge von 0,1 bis 5 Gew.-%. Die Mengenangaben beziehen sich jeweils auf die gesamte wässrige Zusammensetzung. In einer bevorzugten Ausführung enthält die wässrige Zusammensetzung 0 bis 5 Gew.-% 1,2-Propylenglykol und/oder 0 bis 5 Gew.-% Harnstoff.

Eine trockene keratinhaltige Faser gemäß Schritt (iii) des erfindungsgemäßen Verfahrens liegt dann vor, wenn die den Haaren anhaftenden Wasserreste soweit verdampft sind, dass die Haare einzeln fallen. Bevorzugt ist bei einer trockenen keratinhaltigen Faser entweder der Feuchtigkeitsgehalt der Faser mit der Feuchtigkeit der Luft im wesentlichen im Gleichgewicht oder die Faser nimmt Feuchtigkeit aus der Luft der Umgebung auf. Eine solche trockene Faser wird bevorzugt durch Trocknung der nassen Faser mit heißer Luft unter Benutzung eines Föns erzielt. Die Trocknung im Schritt (iii) wird bevorzugt dann ausgeführt, wenn zwischen Schritt (iii) und Schritt (iv) im Rahmen eines Glättungsverfahrens eine Wärmebehandlung während eines zusätzlichen Glättungsschritts, z.B. mit entsprechend temperierten Platten, erfolgt.

In einer weiteren Ausführungsform werden im Rahmen einer Haarglättung die Fasern nach Schritt (iii) einer Wärmebehandlung unter mechanischer Glättung der Faser bei einer Temperatur von 120-220°C unterworfen. Diese Wärmebehandlung unter mechanischer Glättung der Faser findet bevorzugt bei einer Temperatur von 140-200°C statt. Die Wärmebehandlung kann mit heißer Luft erfolgen. In diesem Fall, wird die Faser während des Kämmens genau an der Stelle erwärmt, an der die mechanische Glättung erfolgt. Darüber hinaus ist es besonders bevorzugt, dass die Wärmebehandlung nach Manier des Glättens mit Hilfe entsprechend temperierter Platten, insbesondere Metall- oder Keramikplatten, erfolgt, in dem die Platte auf die zu glättende Faser gedrückt wird und die an die Faser gedrückte Platte entlang der Faser bewegt wird. Die Platten können gegebenenfalls mit hitzebeständigen Werkstoffen beschichtet sein. Besonders bevorzugt wird die zu glättende keratinhaltige Faser zwischen zwei entsprechend temperierte Platten gepreßt und beide Platten zugleich entlang der längsten räumlichen Ausdehnung der Faser bewegt. Dabei ist es wiederum bevorzugt, dass beide Platten miteinander verbunden sind, so dass beide Platten gleichmäßig entlang der Faser bewegt werden können. Wird die Wärmebehandlung am lebenden Haar durchgeführt, so ist die Faser an einem Ende (Haarwurzel) befestigt. Die Platten werden in diesem Fall bevorzugt gleichmäßig von der Haarwurzel weg entlang der gesamten Faser bewegt. Durch diese Bewegung erfolgt eine mechanische Glättung der Faser. Ein entsprechendes Gerät zur Wärmebehandlung ist beispielsweise das Gerät "Ceramic Flat-Master" (Vertrieben durch: Efalock, Deutschland).

Ferner können die Fasern zwischen Schritt (iii) und (iv) und/oder im Rahmen einer Nachbehandlung am Ende des erfindungsgemäßen Verfahrens mit einem handelsüblichen Conditioner behandelt werden.

Ein dritter Gegenstand der Erfindung ist die Verwendung der Mittel des ersten Erfindungsgegenstandes zur Fixierung von umgeformten, insbesondere geglätteten, keratinhaltigen Fasern, insbesondere menschlichen Haaren.

Ein vierter Gegenstand der Erfindung ist ein Kit-of-parts, enthaltend
(a) ein Mittel des ersten Erfindungsgegenstandes,
(b) eine wäßrige Zusammensetzung, enthaltend mindestens eine keratinreduzierende Verbindung,
wobei jede der Zusammensetzungen aus (a) und (b) in einem separaten Container verpackt ist und
(c) gegebenenfalls
   (i) ein oder mehrere Sicherheitsmaterialien zur Vermeidung des unerwünschten Inkontakttretens der Zubereitungen mit dem menschlichen Körper, vorzugsweise Handschuhe und/oder
   (ii) ein oder mehrere Behandlungsmittel in Form eines Conditioners.

Das erfindungsgemäße Kit-of-parts stellt die Mittel zur Durchführung des erfindungsgemäßen Verfahrens des zweiten Erfindungsgegenstandes zur Verfügung.

Die folgenden Beispiele sollen den Gegenstand der Erfindung näher erläutern:

### Beispiele

Es wurden die Fixiermittel gemäß Tabelle 1 nach einem Standardverfahren hergestellt. Die Mittel F1 bis F4 sind erfindungsgemäß. Die Mittel V1 und V2 sind nicht erfindungsgemäß. Ferner wurde nach bekannter Vorgehensweise eine Glättungscreme gemäß Tabelle 2 bereitgestellt.

**Tabelle 1: Fixiermittel**

| | Fixiermittel Nr. | | | | | |
|---|---|---|---|---|---|---|
| Rohstoff | F1 [Gew.%] | F2 [Gew.%] | F3 [Gew.%] | F4 [Gew.%] | V1 [Gew.%] | V2 [Gew.%] |
| Cetearylalkohol | 4,00 | 4,00 | 4,00 | 4,00 | 4,00 | 4,00 |
| Eumulgin^{®} B3¹ | 0,50 | 0,50 | 0,50 | 0,50 | 0,50 | 0,50 |
| Ammoniak (25 %ige wäßrige Lösung) | 0,80 | 0,80 | 0,80 | 0,80 | 0,80 | 0,80 |
| Dipicolinsäure | 0,10 | 0,10 | 0,10 | 0,10 | 0,10 | 0,10 |
| Turpinal^{®} SL² | 1,70 | 1,70 | 1,70 | 1,70 | 1,70 | 1,70 |
| Rheocare^{®} CTH(E)³ | 1,00 | 1,00 | 1,00 | 1,00 | - | 1,00 |
| Dehyquart^{®} A CA⁴ | 2,00 | 1,00 | 1,00 | 2,00 | - | - |
| Merquat^{®} 100⁵ | - | - | - | 1,00 | - | - |
| Dehyquart^{®} E⁶ | 1,00 | - | 1,00 | 1,00 | - | - |
| Dehyquart^{®} L80⁷ | - | 1,00 | - | - | - | - |
| Dow Corning^{®} 1403⁸ | 1,00 | 1,00 | - | 1,00 | - | - |
| Wasserstoffperoxid (50 %ige wäßrige Lösung) | 4,00 | 4,00 | 4,00 | 4,00 | 4,00 | 4,00 |
| Wasser | ad 100 | ad 100 | ad 100 | ad 100 | ad 100 | ad 100 |

| | | | | | | |
|---|---|---|---|---|---|---|
| ¹Cetylstearylalkohol, ethoxyliert mit 30 Einheiten Ethylenoxid (INCI-Bezeichnung: Ceteareth-30) (Cognis) ² 1-Hydroxyethan-1,1-diphosphonsäure (INCI-Bezeichnung: Etidronic Acid, Aqua (Water)) (Solutia) ³ Trimethylammonioethyl methacrylat Chlorid Homopolymer, (INCI-Bezeichnung: Polyquaternium-37, Propylene Glycol Dicaprylate/Dicaprate, PPG-1 Trideceth-6) (CRL Cosmetic Rheologies, Ltd.) ⁴ Poly(dimethyldiallylammoniumchlorid) (INCI-Bezeichnung: Polyquaternium-6) (Nalco) ⁵ Trimethylhexadecylammoniumchlorid, 25% Aktivsubstanz (INCI-Bezeichnung: Aqua (Water), Cetrimonium Chloride) (Cognis) ⁶ N-(2-Hydroxyhexadecyl)-N-2-hydroxyethyl-N,N-dimethylammonium Chlorid (23% Aktivsubstanz, INCI-Bezeichnung: Aqua (Water), Hydroxycetyl Hydroxyethyl Dimonium Chloride) (Cognis) ⁷ N,N-Di(Cocoylethyl-N-hydroxyethyl-N-methylammonium Methylsulfat (77% Aktivsubstanz; INCI-Bezeichnung: Dicocoylethyl Hydroxyethylmonium Methosulfate, Propylene Glycol) (Cognis) ⁸ INCI-Bezeichnung: Dimethicone, Dimethiconol (Dow Corning) | | | | | | |

**Tabelle 2: Glättungscreme**

| Rohstoff | [Gew.%] |
|---|---|
| 1,2-Propylenglykol | 2,00 |
| Cetyl/Stearylalkohol | 9,00 |
| Lanette^{®} E⁹ | 0,50 |
| Brij^{®} 35P¹⁰ | 0,50 |
| Natrosol^{®} 250 HR¹¹ | 0,25 |
| Ammoniak (25%ige wäßrige Lösung | 5,00 |
| Turpinal^{®} SL² | 0,25 |
| Ammoniumthioglykolat (71 %ige wäßrige Lösung) | 18,00 |
| Ammoniumbicarbonat | 4,00 |
| Promois Silk^{®} 1000¹² | 1,00 |
| Dow Corning^{®} 1403⁸ | 0,50 |
| Parfüm | 1,00 |
| Wasser | ad 100 |

| | |
|---|---|
| ⁹ Sodium Cetearyl Sulfate (Cognis) ¹⁰ Polyethylenglykolmonolaurylether mit 23 Einheiten Ethylenoxid (INCI-Bezeichnung: Laureth-23) (Uniqema) ¹¹ Hydroxyethylcellulose (Hercules) ¹² Collagen Hydrolysat (INCI-Bezeichnung: Hydrolyzed Silk) (RITA Corp.) | |

### Versuchsdurchführung und Beurteilung der Ergebnisse:

Es wurde eine Gruppe von Testpersonen ausgewählt, deren Mitglieder vor Beginn der Durchführung des Glättungsverfahrens vergleichbar stark gelocktes Haar mit einem identischen Schädigungsgrad besaßen.

Das gesamte Kopfhaar einer Testperson wurde mit Wasser angefeuchtet und mit einem Handtuch frottiert.

Das gesamte Haar wurde mit der obenstehenden Glättungscreme behandelt und glatt gekämmt. Nach einer Einwirkungszeit von 20 Minuten wurde das Haar gespült und vorsichtig mit einem Handtuch frottiert. Das Haar wurde sodann mit einem der Fixiermittel gemäß Tabelle 1 behandelt und erneut glatt gekämmt. Nach einer Einwirkungszeit von 15 Minuten wurde das Haar mit Wasser gespült und getrocknet.

Dieses Verfahren wurde mit allen Fixiermitteln F1 bis F4, V1 und V2 der Tabelle 1 durchlaufen. Je Fixiermittel wurde eine Testperson benötigt.

Die Beurteilung des Glättungsergebnisses und der Kämmbarkeit geschah durch sechs fachkundige Personen. Diesen fachkundigen Personen wurde mitgeteilt, welche der Testpersonen miteinander verglichen werden sollten. Es wurde jedoch nicht bekanntgegeben, welche dieser Testpersonen erfindungsgemäß behandelt wurde und welche nicht.

Der Bewertungsmaßstab war:
++ viel besser als Vergleichstestperson
+ besser als Vergleichstestperson
◇ genau wie Vergleichstestperson
- schlechter als Vergleichstestperson
-- viel schlechter als Vergleichstestperson

Die gemittelten Ergebnisse sind Tabelle 3 zu entnehmen.

| Tabelle 3: Bewertung | Strähne mit Fixiermittel F behandelt: | | | |
|---|---|---|---|---|
| | F1 | F2 | F3 | F4 |
| Glättungsergebnis gegenüber V1 | ++ | ++ | ++ | ++ |
| Glättungsergebnis gegenüber V2 | + | + | + | + |
| Kämmbarkeit gegenüber V1 | ++ | ++ | ++ | ++ |
| Kämmbarkeit gegenüber V2 | + | + | + | + |

## Patentansprüche

1. Mittel enthaltend mindestens ein Oxidationsmittel sowie eine Wirkstoffkombination aus den Komponenten
(a) einem kationisches Polymer, das mindestens ein Strukturelement der allgemeinen Formel (I) besitzt, 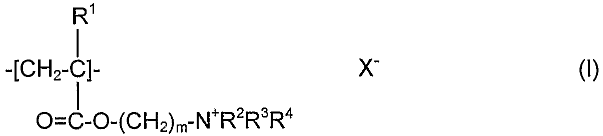 in der
- R¹ ein Wasserstoffatom oder eine (C₁- bis C₄)-Alkylgruppe ist,
- R², R³ und R⁴ unabhängig voneinander stehen für (C₁- bis C₄)-Alkyl-gruppen, (C₂- bis C₄)-Alkenylgruppen oder (C₂- bis C₄)-Hydroxyalkylgruppen,
- m eine Zahl 1, 2, 3 oder 4 ist und
- X ein physiologisch verträgliches organisches oder anorganisches Anion ist,
(b) mindestens einer quartären Ammoniumverbindung der Formel (II)
(R⁵)_{y}(Me)_{(4-y)}N⁺ Y⁻ (II)
wobei
- R⁵ eine lineare oder verzweigte (C₈- bis C₃₀)-Alkylgruppe,
- y gleich 1 oder 2 ist und
- Y⁻ ein physiologisch verträgliches organisches oder anorganisches Anion ist, und
(c) mindestens einer quartären Ammoniumverbindung, welche mindestens eine Gruppe der Formel (III) enthält,
R⁶-A¹-A²-A³- (III),
wobei
- R⁶ für eine gesättigte oder ungesättigte, lineare oder verzweigte, gegebenenfalls substituierte (C₈- bis C₃₀)-Kohlenwasserstoffgruppe steht,
- A¹ für eine Carbonylgruppe oder eine direkte Bindung steht,
- A² für eine direkte Bindung, NH oder ein Sauerstoffatom steht und
- A³ eine (C₂- bis C₄)-Alkylengruppe oder eine (C₂- bis C₄)-Hydroxyalkylengruppe bedeutet, mit den Maßgaben, dass
A³ an das quartäre Stickstoffatom der quartären Ammoniumverbindung bindet und
A³ eine (C₂- bis C₄)-Hydroxyalkylengruppe bedeutet, wenn A¹ und A² für eine direkte Bindung stehen.

2. Mittel nach Anspruch 1 **dadurch gekennzeichnet, dass** das Oxidationsmittel Wasserstoffperoxyd ist.

3. Mittel nach einem der Ansprüche 1 oder 2, **dadurch gekennzeichnet, dass** das Oxidationsmittel in einer Menge von 0,5 bis 3,0 Gew.-%, bezogen auf das Gewicht des Mittels, enthalten ist.

4. Mittel nach einem der Ansprüche 1 bis 3, **dadurch gekennzeichnet, dass** das kationische Polymer, das mindestens ein Strukturelement gemäß Formel (I) besitzt, ein Homopolymer dieses Strukturelements ist.

5. Mittel nach einem der Ansprüche 1 bis 4, **dadurch gekennzeichnet, dass** das Polymer in einer Menge von 0,01 bis 10 Gew.-% bezogen auf das Gewicht des Mittels enthalten ist.

6. Mittel nach einem der Ansprüche 1 bis 5, **dadurch gekennzeichnet, dass** die Verbindung gemäß Formel (II) ausgewählt wird aus Salzen des Dicetyldimethylammoniums, des Cetyltrimethylammoniums, des Distearyldimethylammoniums oder des Stearyltrimethylammoniums.

7. Mittel nach einem der Ansprüche 1 bis 6, **dadurch gekennzeichnet, dass** das Stoffmengenverhältnis der Komponenten (a) zu (b) im Bereich von 0,2 zu 1 bis 5 zu 1 liegt.

8. Mittel nach einem der Ansprüche 1 bis 7, **dadurch gekennzeichnet, dass** das Stoffmengenverhältnis der Komponenten (a) zu (c) im Bereich von 0,2 zu 1 bis 5 zu 1 liegt.

9. Mittel nach einem der Ansprüche 1 bis 8, **dadurch gekennzeichnet, dass** die quartären Ammoniumverbindungen (c) in einer Menge von 0,05 bis 5 Gew.-% bezogen auf das Gewicht des Mittels enthalten sind.

10. Mittel nach einem der Ansprüche 1 bis 9, **dadurch gekennzeichnet, dass** die quartären Ammoniumverbindungen (c) aus der Gruppe von quartären Ammoniumverbindungen ausgewählt werden, die eine oder zwei Gruppen gemäß Formel (III) tragen.

11. Mittel nach einem der Ansprüche 1 bis 10, **dadurch gekennzeichnet, dass** der Rest R⁶ gemäß Formel (III) steht für eine lineare oder verzweigte (C₈- bis C₃₀)-Alkylgruppe, lineare oder verzweigte (C₈- bis C₃₀)-Alkenylgruppe, eine Hydroxy-(C₈- bis C₃₀)-alkylgruppe oder eine Oligohydroxy-(C₈- bis C₃₀)-alkylgruppe.

12. Mittel nach einem der Ansprüche 1 bis 10, **dadurch gekennzeichnet, dass** die Gruppe gemäß Formel (III) ausgewählt wird aus einer ω-[(C₈- bis C₃₀)-Alkanoyloxy]-(C₂- bis C₄)-alkylgruppe, einer ω-[(C₈- bis C₃₀)-Alkylamido]-(C₂- bis C₄)-alkylgruppe, oder einer (C₁₀- bis C₃₆)-Hydroxyalkylgruppe.

13. Mittel nach einem der Ansprüche 1 bis 10, **dadurch gekennzeichnet, dass** die quartäre Ammoniumverbindung (c) des Anspruchs 1 ausgewählt wird aus Verbindungen gemäß Formel (c1) 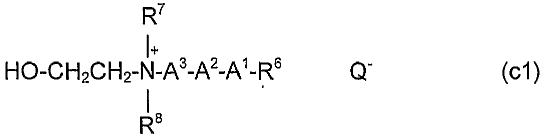 worin
- R⁷ und R⁸ unabhängig voneinander ein Wasserstoffatom, eine (C₂- bis C₃₀)-Acylgruppe oder eine lineare oder verzweigte (C₁- bis C₃₀)-Alkylgruppe bedeutet,
- R⁶ eine lineare oder verzweigte (C₈- bis C₃₀)-Alkylgruppe oder eine lineare oder verzweigte (C₆- bis C₃₀)-Alkenylgruppe bedeutet,
- A¹ eine direkte Bindung oder eine Carbonylgruppe bedeutet,
- A² eine direkte Bindung, eine Gruppe NH oder ein Sauerstoffatom bedeutet,
- A³ eine (C₂- bis C₄)-Alkylengruppe oder eine (C₂- bis C₄)-Hydroxy-alkylengruppe bedeutet und
- Q⁻ ein physiologisch verträgliches organisches oder anorganisches Anion ist, mit der Maßgabe, dass A³ eine (C₂- bis C₄)-Hydroxyalkylengruppe bedeutet, wenn A¹ und A² für eine direkte Bindung stehen.

14. Mittel nach einem der Ansprüche 1 bis 10, **dadurch gekennzeichnet, dass** die quartäre Ammoniumverbindung (c) des Anspruchs 1 ausgewählt wird aus Verbindungen gemäß Formel (c2) 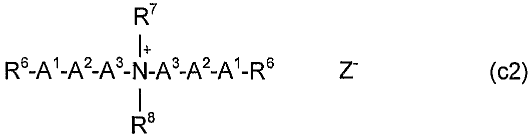 worin
- R⁷ und R⁸ unabhängig voneinander für ein Wasserstoffatom, eine (C₁- bis C₄)-Alkylgruppe oder (C₂- bis C₄)-Hydroxyalkylgruppe stehen,
- R⁶ eine lineare oder verzweigte (C₈- bis C₃₀)-Alkylgruppe, lineare oder verzweigte (C₈- bis C₃₀)-Alkenylgruppe, eine Hydroxy-(C₈- bis C₃₀)-alkylgruppe oder eine Oligohydroxy-(C₈- bis C₃₀)-alkylgruppe bedeutet,
- A¹ eine Carbonylgruppe bedeutet
- A² eine Gruppe NH oder ein Sauerstoffatom bedeutet,
- A³ eine (C₂- bis C₄)-Alkylengruppe, eine (C₂- bis C₄)-Hydroxyalkylengruppe bedeutet und
- Z⁻ ein physiologisch verträgliches organisches oder anorganisches Anion ist.

15. Mittel nach einem der Ansprüche 1 bis 14, **dadurch gekennzeichnet, dass** es zusätzlich mindestens ein Silikon enthält.

16. Verfahren zur Umformung von keratinhaltigen Fasern, insbesondere menschlichen Haaren, **dadurch gekennzeichnet, dass**
(i) die Fasern unter Zuhilfenahme von Verformungshilfsmitteln nach, vor oder während Schritt (ii) verformt werden,
(ii) eine wäßrige Zusammensetzung, enthaltend mindestens eine keratinreduzierende Verbindung, auf die Fasern aufgetragen wird,
(iii) die Fasern nach einer Einwirkzeit Z1 gespült und gegebenenfalls getrocknet werden,
(iv) anschließend ein Mittel gemäß einem der Ansprüche 1 bis 15 auf die geglätteten Fasern aufgetragen und nach einer Einwirkzeit Z2 wieder abgespült wird.

17. Verfahren nach Anspruch 16, **dadurch gekennzeichnet, dass** die keratinhaltigen Fasern vor dem Schritt (i) angefeuchtet werden.

18. Verwendung mindestens eines Mittels gemäß einem der Ansprüche 1 bis 15 zur Fixierung von umgeformten keratinhaltigen Fasern, insbesondere menschlichen Haaren.

19. Kit-of-parts enthaltend
(a) ein Mittel gemäß einem der Ansprüche 1 bis 15,
(b) eine wäßrige Zusammensetzung, enthaltend mindestens eine keratinreduzierende Verbindung,
wobei jede der Zusammensetzungen aus (a) und (b) in einem separaten Container verpackt ist und
(c) gegebenenfalls
(i) ein oder mehrere Sicherheitsmaterialien zur Vermeidung des unerwünschten Inkontakttretens der Zubereitungen mit dem menschlichen Körper, vorzugsweise Handschuhe und/oder
(ii) ein oder mehrere Behandlungsmittel in Form eines Conditioners.

## Claims

1. An agent containing at least one oxidizing agent and an active substance combination comprising the components
(a) a cationic polymer, which has at least one structural element of the general formula (I) 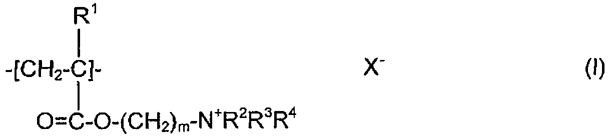 in which
- R¹ is a hydrogen atom or a (C₁ to C₄) alkyl group,
- R², R³, and R⁴ independently of one another stand for (C₁ to C₄) alkyl groups, (C₂ to C₄) alkenyl groups, or (C₂ to C₄) hydroxyalkyl groups,
- m is a number 1, 2, 3, or 4, and
- X⁻ is a physiologically acceptable organic or inorganic anion,
(b) at least one quaternary ammonium compound of the formula (II)
(R⁵)_{y}(Me)_{(4-y)}N⁺ Y⁻ (II)
whereby
- R⁵ is a linear or branched (C₈ to C₃₀) alkyl group,
- y is 1 or 2, and
- Y⁻ is a physiologically acceptable organic or inorganic anion, and
(c) at least one quaternary ammonium compound, which contains at least one group of the formula (III),
R⁶-A¹-A²-A³- (III),
whereby
- R⁶ stands for a saturated or unsaturated, linear or branched, optionally substituted (C₈ to C₃₀) hydrocarbon group,
- A¹ stands for a carbonyl group or a direct bond,
- A² stands for a direct bond, NH, or an oxygen atom, and
- A³ denotes a (C₂ to C₄) alkylene group or a (C₂ to C₄) hydroxyalkylene group, with the provisos that
A³ binds to the quaternary nitrogen atom of the quaternary ammonium compound, and
A³ denotes a (C₂ to C₄) hydroxyalkylene group, if A¹ and A² stand for a direct bond.

2. The agent according to claim 1, **characterized in that** the oxidizing agent is hydrogen peroxide.

3. The agent according to one of claims 1 or 2, **characterized in that** the oxidizing agent is present in an amount of 0.5 to 3.0% by weight, based on the weight of the agent.

4. The agent according to one of claims 1 to 3, **characterized in that** the cationic polymer, which has at least one structural element according to formula (I), is a homopolymer of said structural element.

5. The agent according to one of claims 1 to 4, **characterized in that** the polymer is present in an amount of 0.01 to 10% by weight, based on the weight of the agent.

6. The agent according to one of claims 1 to 5, **characterized in that** the compound according to formula (II) is selected from the salts of dicetyldimethylammonium, cetyltrimethylammonium, distearyldimethylammonium, or stearyltrimethylammonium.

7. The agent according to one of claims 1 to 6, **characterized in that** the molar ratio of the components (a) to (b) is in the range of 0.2 to 1 to 5 to 1.

8. The agent according to one of claims 1 to 7, **characterized in that** the molar ratio of the components (a) to (c) is in the range of 0.2 to 1 to 5 to 1.

9. The agent according to one of claims 1 to 8, **characterized in that** the quaternary ammonium compounds (c) are present in an amount of 0.05 to 5% by weight, based on the weight of the agent.

10. The agent according to one of claims 1 to 9, **characterized in that** the quaternary ammonium compounds (c) are selected from the group of quaternary ammonium compounds that carry one or two groups according to the formula (III).

11. The agent according to one of claims 1 to 10, **characterized in that** the group R⁶ according to the formula (III) stands for a linear or branched (C₈ to C₃₀) alkyl group, linear or branched (C₈ to C₃₀) alkenyl group, a hydroxy-(C₈ to C₃₀)-alkyl group, or an oligohydroxy-(C₈ to C₃₀)-alkyl group.

12. The agent according to one of claims 1 to 10, **characterized in that** the group according to the formula (III) is selected from an ω-[(Ca to C₃₀)-alkanoyloxy]-(C₂ to C₄)-alkyl group, an ω-[(C₈ to C₃₀)-alkylamido]-(C₂ to C₄)-alkyl group, or a (C₁₀ to C₃₆) hydroxyalkyl group.

13. The agent according to one of claims 1 to 10, **characterized in that** the quaternary ammonium compound (c) of claim 1 is selected from compounds according to the formula (c1) 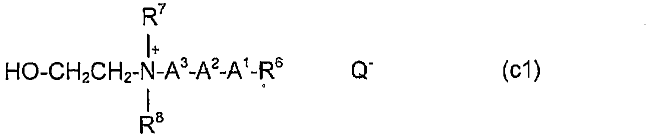 where
- R⁷ and R⁸ independently of another denote a hydrogen atom, a (C₂ to C₃₀) acyl group, or a linear or branched (C₁ to C₃₀) alkyl group,
- R⁶ denotes a linear or branched (C₈ to C₃₀) alkyl group or a linear or branched (C₈ to C₃₀) alkenyl group,
- A¹ denotes a direct bond or a carbonyl group,
- A² denotes a direct bond, an NH group, or an oxygen atom,
- A³ denotes a (C₂ to C₄) alkylene group or a (C₂ to C₄) hydroxyalkylene group, and
- Q⁻ is a physiologically acceptable organic or inorganic anion, with the proviso that A³ denotes a (C₂ to C₄) hydroxyalkylene group, if A¹ and A² stand for a direct bond.

14. The agent according to one of claims 1 to 10, **characterized in that** the quaternary ammonium compound (c) of claim 1 is selected from compounds according to the formula (c2) 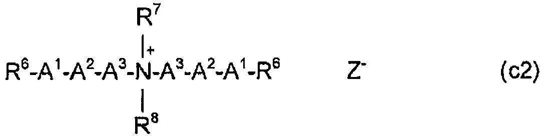 where
- R⁷ and R⁸ independently of one another stand for a hydrogen atom, a (C₁ to C₄) alkyl group, or (C₂ to C₄) hydroxyalkyl group,
- R⁶ denotes a linear or branched (C₈ to C₃₀) alkyl group, linear or branched (C₈ to C₃₀) alkenyl group, a hydroxy-(C₈ to C₃₀)-alkyl group, or an oligohydroxy-(C₈ to C₃₀)-alkyl group,
- A¹ denotes a carbonyl group,
- A² denotes an NH group or an oxygen atom,
- A³ denotes a (C₂ to C₄) alkylene group, a (C₂ to C₄) hydroxyalkylene group, and
- Z⁻ is a physiologically acceptable organic or inorganic anion.

15. The agent according to one of claims 1 to 14, **characterized in that** it contains in addition at least one silicone.

16. A method for reshaping keratin-containing fibers, especially human hair, **characterized in that**
i. the fibers are shaped with the help of shaped aids after, before, or during step (ii),
ii. an aqueous composition, containing at least one keratin-reducing compound, is applied to the fibers,
iii. the fibers are rinsed and optionally dried after a treatment time Z1,
iv. then an agent according to one of claims 1 to 15 is applied to the smoothed fibers and rinsed off again after a treatment time Z2.

17. The method according to claim 16, **characterized in that** the keratin-containing fibers are moistened before step (i).

18. Use of at least one agent according to one of claims 1 to 15 for fixing reshaped, keratin-containing fibers, especially human hair.

19. A kit-of-parts containing
a) an agent according to one of claims 1 to 15,
b) an aqueous composition, containing at least one keratin-reducing compound, whereby each of the compositions of (a) and (b) is packaged in a separate container and
c) optionally
i. one or more safety materials to prevent undesirable contact of the preparations with the human body, preferably gloves, and/or
ii. one or more treatment agents in the form of a conditioner.

## Revendications

1. Agent contenant au moins un agent d'oxydation et une combinaison de substances actives constituée des composants suivants :
(a) un polymère cationique qui possède au moins un élément structurel de la formule générale (I), 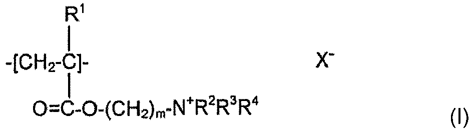 dans laquelle
- R¹ est un atome d'hydrogène ou un groupe alkyle en C₁ à C₄,
- R², R³ et R⁴ sont indépendamment des groupes alkyles en C₁ à C₄, des groupes alcényle en C₂ à C₄ ou des groupes hydroxyalkyle en C₂ à C₄,
- m est un nombre égal à 1, 2, 3 ou 4 et
- X⁻ est un anion organique ou minéral physiologiquement compatible,
(b) au moins un composé d'ammonium quaternaire de la formule (II)
(R⁵)_{y}(Me)_{(4-y)}N⁺ Y⁻ (II)
dans laquelle
- R⁵ est un groupe alkyle en C₈ à C₃₀ linéaire ou ramifié,
- y vaut 1 ou 2 et
- Y⁻ est un anion organique ou minéral physiologiquement compatible, et
(c) au moins un composé d'ammonium quaternaire qui contient au moins un groupe de la formule (III),
R⁶-A¹-A²-A³ (III),
dans laquelle
- R⁶ représente un groupe hydrocarbure en C₈ à C₃₀, saturé ou insaturé, linéaire ou ramifié, éventuellement substitué,
- A¹ représente un groupe carbonyle ou une liaison directe,
- A² représente une liaison directe, NH ou un atome d'oxygène, et
- A³ est un groupe alkylène en C₂ à C₄ ou un groupe hydroxyalkylène en C₂ à C₄, sous réserve que
A³ se lie à l'atome d'azote quaternaire du composé d'ammonium quaternaire et
A³ un groupe hydroxyalkylène en C₂ à C₄ lorsque A¹ et A² représente une liaison directe.

2. Agent selon la revendication 1, **caractérisé en ce que** l'agent d'oxydation est le peroxyde d'hydrogène.

3. Agent selon l'une des revendications 1 ou 2, **caractérisé en ce que** l'agent d'oxydation est contenu dans une quantité allant de 0,5 à 3,0% en poids, sur la base du poids de l'agent.

4. Agent selon l'une des revendications 1 à 3, **caractérisé en ce que** le polymère cationique, qui possède au moins un élément structurel de formule (I), est un homopolymère de cet élément structurel.

5. Agent selon l'une des revendications 1 à 4, **caractérisé en ce que** le polymère est contenu dans une quantité allant de 0,01 à 10% en poids sur la base du poids de l'agent.

6. Agent selon l'une des revendications 1 à 5, **caractérisé en ce que** le composé de formule (II) est choisi parmi les sels du dicétyldiméthylammonium, du cétyltriméthylammonium, du distéaryldiméthylammonium ou du stéaryltriméthylammonium.

7. Agent selon l'une des revendications 1 à 6, **caractérisé en ce que** le rapport molaire des composants (a) à (b) est dans la gamme allant de 0,2:1 à 5:1.

8. Agent selon l'une des revendications 1 à 7, **caractérisé en ce que** le rapport molaire des composants (a) à (c) est dans la gamme allant de 0,2:1 à 5:1.

9. Agent selon l'une des revendications 1 à 8, **caractérisé en ce que** les composés d'ammonium quaternaire (c) sont contenus dans une quantité allant de 0,05 à 5% en poids sur la base du poids de l'agent.

10. Agent selon l'une des revendications 1 à 9, **caractérisé en ce que** les composés d'ammonium quaternaire (c) sont choisis dans le groupe des composés d'ammonium quaternaire qui portent un ou deux groupes de formule (III).

11. Agent selon l'une des revendications 1 à 10, **caractérisé en ce que** le radical R⁶ de formule (III) représente un groupe alkyle en C₈ à C₃₀ linéaire ou ramifié, un groupe alcényle en C₈ à C₃₀ linéaire ou ramifié, un groupe hydroxyalkyle en C₈ à C₃₀ ou un groupe oligohydroxyalkyle en C₈ à C₃₀.

12. Agent selon l'une des revendications 1 à 10, **caractérisé en ce que** le groupe de formule (III) est sélectionné parmi un groupe ω -[alcanoyloxy en C₈ à C₃₀]-(alkyle en C₂ à C₄), un groupe ω-[alkylamido en C₈ à C₃₀]-alkyle en C₂ à C₄, ou un groupe hydroxyalkyle en C₁₀ à C₃₆.

13. Agent selon l'une des revendications 1 à 10, **caractérisé en ce que** le composé d'ammonium quaternaire (c) de la revendication 1 est choisi parmi les composés de formule (c1) 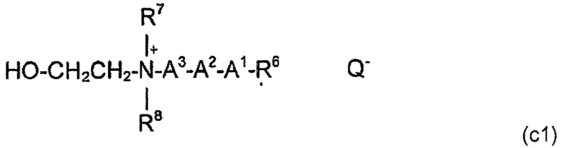 dans laquelle
- R⁷ et R⁸ sont indépendamment un atome d'hydrogène, un groupe acyle en C₂ à C₃₀, un groupe alkyle en C₁ à C₃₀ linéaire ou ramifié,
- R⁶ est un groupe alkyle C₈ à C₃₀ linéaire ou ramifié ou un groupe alcényle C₈ à C₃₀ linéaire ou ramifié,
- A¹ est une liaison directe ou un groupe carbonyle,
- A² est une liaison directe, un groupe NH ou un atome d'oxygène,
- A³ un groupe alkylène en C₂ à C₈ ou un groupe hydroxyalkylène en C₂ à C₈ et
- Q⁻ est un anion organique ou minéral physiologiquement compatible, sous réserve que A³ est un groupe hydroxyalkyle en C₂ à C₄ si A¹ et A² sont une liaison directe.

14. Agent selon l'une des revendications 1 à 10, **caractérisé en ce que** le composé d'ammonium quaternaire (c) de la revendication 1 est choisi parmi les composés de formule (c2) 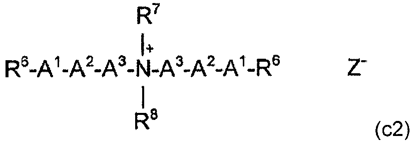 dans laquelle
- R⁷ et R⁸ sont chacun indépendamment un atome d'hydrogène, un groupe alkyle en C₁ à C₄ ou un groupe hydroxyalkyle en C₂ à C₄,
- R⁶ est un groupe alkyle en C₈ à C₃₀ linéaire ou ramifié, un groupe alcényle en C₈ à C₃₀ linéaire ou ramifié, un groupe hydroxyalkyle en C₈ à C₃₀ ou un groupe oligohydroxyalkyle en C₈ à C₃₀,
- A¹ représente un groupe carbonyle,
- A² est un groupe NH ou un atome d'oxygène,
- A³ représente un groupe alkylène en C₂ à C₄, un groupe hydroxyalkylène en C₂ à C₄ et
- Z⁻ est un anion organique ou minéral physiologiquement compatible.

15. Agent selon l'une des revendications 1 à 14, **caractérisé en ce qu'**elle contient en outre au moins une silicone.

16. Procédé de mise en forme de fibres contenant de la kératine, notamment des cheveux humains, **caractérisé en ce que**
(i) les fibres sont mise en forme à l'aide d'outils de mise en forme après, avant ou pendant l'étape (ii),
(ii) une composition aqueuse contenant au moins un composé réducteur de kératine est appliquée sur les fibres,
(iii) les fibres sont rincées au bout d'un temps d'action Z1 et éventuellement séchées,
(iv) puis un agent selon l'une des revendications 1 à 15 est appliqué sur les fibres lissées et de nouveau rincé au bout d'un temps d'action Z2.

17. Procédé selon la revendication 16, **caractérisé en ce que** les fibres contenant de la kératine sont humidifiées avant l'étape (i).

18. Utilisation d'au moins un agent selon l'une des revendications 1 à 15 pour fixer des fibres contenant de la kératine, en particulier des cheveux humains, qui ont été mises en forme.

19. Kit d'éléments comprenant
(a) un agent selon l'une des revendications 1 à 15,
(b) une composition aqueuse contenant au moins un composé réducteur de kératine,
chacune desdites compositions en (a) et (b) étant conditionnée dans un récipient séparé et
(c) éventuellement
(i) un ou plusieurs matériau de protection servant à empêcher le contact indésirable des préparations avec le corps humain, de préférence des gants et/ou
(ii) un ou plusieurs agents de traitement se présentant sous la forme d'un conditionneur.
